# EUROPEAN PATENT APPLICATION

(11) **EP 4 406 565 A1**
(43) Date of publication of application: **31.07.2024**
(21) Application number: 24153308.2
(22) Date of filing: 23.01.2024
(51) Int. Cl.: A61M 1/38, A61M 1/36

(54) **PACKAGING TRAY FOR A BLOOD PROCESSING KIT**

(30) Priority: 25.01.2023 US 202363481524 P
(71) Applicant: Fenwal, Inc., Lake Zurich, IL 60047 (US)
(72) Inventor: KUSTERS, Benjamin E., Lake Zurich, 60047 (US); MADSEN, James Gregory, Lake Zurich, 60047 (US); MIN, Kyungyoon, Lake Zurich, 60047 (US)
(74) Representative: Maikowski & Ninnemann Patentanwälte Partnerschaft mbB

(57) **Abstract**

A packaging tray for use in a blood processing system including a blood processing device having device components arranged on a component surface and a removable fluid flow circuit having circuit components configured to interact with corresponding device components, wherein the packaging tray configured to be selectively arranged on and removable from the component surface. The packaging tray includes a first surface and a second surface opposite to the first surface and spaced apart by a thickness, component sections arranged at positions corresponding to positions of respective device components, the component sections respectively formed, at least partially, as openings extending through the thickness, and a plurality of recessed pathways formed on the first surface, the recessed pathways extending between the component sections. A blood processing device may include the packaging tray, and a blood processing system may include a blood processing device and the packaging tray.

## Description

### Cross-Reference to Related Application(s)

This application claims the benefit of and priority to U.S. Provisional Patent Application Serial No. 63/481,524 filed on January 25, 2023, the contents of which are incorporated herein by reference.

### Field of the Disclosure

The present disclosure relates to a blood processing system and/or a blood component collecting or manufacturing system having a reusable blood processing device and a disposable fluid flow circuit. More particularly, the present disclosure relates to a blood processing system further including a packaging tray on which components of the fluid flow circuit can be arranged such that the components of the fluid flow circuit can interface with corresponding components of the processing device when the packaging tray is positioned on the processing device.

### Description of Related Art

Various blood processing systems now make it possible to collect particular blood constituents, instead of whole blood, from a blood source such as, but not limited to, a container of previously collected blood or other living or non-living source. Typically, in such systems, whole blood is drawn from a blood source, the particular blood component or constituent is separated, removed, and collected, and the remaining blood constituents are returned to the blood source. Removing only particular constituents is advantageous when the blood source is a human donor, because potentially less time is needed for the donor's body to return to pre-donation levels, and donations can be made at more frequent intervals than when whole blood is collected. This increases the overall supply of blood constituents, such as plasma and platelets, made available for transfer and/or therapeutic treatment.

Whole blood is typically separated into its constituents (e.g., red cells, platelets, and plasma) through centrifugation, such as in the AMICUS^{®} separator from Fenwal, Inc. of Lake Zurich, III., which is an affiliate of Fresenius Kabi AG of Bad Homburg, Germany, or other centrifugal separation devices, or a spinning membrane-type separator, such as the AUTOPHERESIS-C^{®} and AURORA^{®} devices from Fenwal, Inc.

In a whole blood processing setting, a disposable kit including various fluid containers, conduits and related components is arranged on a reusable processing device by individually aligning and mating the components of the disposable kit with corresponding components of the processing device. In a known system, the fluid containers may be provided in selected combinations including one or more whole blood container, a red blood cell container, a plasma container, a buffy coat container and/or an additive solution container. The containers are individually arranged on corresponding hooks of a rack extending vertically relative to the processing device to a height above the processing device, such that the containers hang from the rack. The disposable kit may also include a cassette from which a number of pumping loops extend. The cassette may also define various flow paths or conduits to fluidically connect various components of the disposable kit. The cassette is aligned with a corresponding nesting module of the processing device and the pumping loops are arranged relative to corresponding pumps on the processing device, such that the pumps operate to promote fluid flow through the pumping loops, and in turn, through the flow paths and/or conduits.

Additional components of the disposable kit, such as a separation chamber and various fluid tubes are also arranged relative to corresponding components of the processing device, such as a centrifuge or other separation device and various clamps and/or valves. In this manner, components of the disposable kit may selectively interact with corresponding components of the processing device to perform a selected blood processing, collecting or manufacturing operation.

While the known system provides for reliable blood processing, collection and/or manufacturing operations, it would be desirable to reduce assembly time of the system when arranging components of the disposable kit relative to corresponding components of the processing device.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a packaging tray for use in a blood processing system comprising a blood processing device having device components arranged on a component surface and a removable fluid flow circuit having circuit components configured to interact with corresponding device components, is configured to be selectively arranged on and removable from the component surface. The packaging tray includes a first surface and a second surface opposite to the first surface and spaced apart by a thickness, component sections arranged at positions corresponding to positions of respective device components, the component sections respectively formed, at least partially, as openings extending through the thickness, and a plurality of recessed pathways formed on the first surface, the recessed pathways extending between the component sections.

In another aspect, a blood processing device includes device components arranged on a component surface, a holder configured to support one or more fluid containers, and a packaging tray configured to be selectively arranged on and removable from the component surface. The packaging tray includes a first surface and a second surface opposite to the first surface and spaced apart by a thickness, component sections arranged at positions corresponding to positions of respective device components, the component sections respectively formed, at least partially, as openings extending through the thickness, and a plurality of recessed pathways formed on the first surface, the recessed pathways extending between the component sections.

In still another aspect, a blood processing system includes a blood processing device, a packaging tray, and a fluid flow circuit. The blood processing device includes device components arranged on a component surface and a holder configured to support one or more fluid containers. The packaging tray is configured to be selectively arranged on and removable from the component surface, and includes a first surface and a second surface opposite to the first surface and spaced apart by a thickness, component sections arranged at positions corresponding to positions of respective device components, the component sections respectively formed, at least partially, as openings extending through the thickness, and a plurality of recessed pathways formed on the first surface, the recessed pathways extending between the component sections. The fluid flow circuit includes circuit components configured to interact with corresponding device components, wherein the one or more fluid containers are arrangable in fluid communication with the circuit components. The component sections are configured to accommodate corresponding circuit components such that the circuit components are interfaced with corresponding device components with the packaging tray arranged on the component surface.

These and other aspects of the present subject matter are set forth in the following detailed description of the accompanying drawings.

### Brief Description of the Drawings

FIG. 1 is a perspective of a blood processing device and selected portions of a fluid flow circuit of a blood processing system and/or blood component collecting or manufacturing system according to an embodiment;
FIG. 2 is a perspective view of the processing device of FIG. 1;
FIG. 3 is a diagram of a fluid flow circuit of a blood processing system and/or blood component collecting or manufacturing system according to an embodiment;
FIG. 4 is a perspective view of a packaging tray of a blood processing system and/or blood component collecting or manufacturing system according to an embodiment;
FIG. 5 is a perspective view showing portions of the fluid flow circuit arranged on the packaging tray;
FIG. 6 is another perspective view showing portions of the fluid flow circuit arranged on the packaging tray;
FIG. 7 is a partially exploded view of the blood processing system and/or blood component collecting or manufacturing system of FIG. 1, illustrating portions of the fluid flow circuit arranged on the packaging tray and spaced from the processing device; and
FIG. 8 is a perspective view of the blood processing system and/or blood component collecting or manufacturing system having components of the fluid flow circuit arranged on the packaging tray and the packaging tray positioned on the processing device to perform a blood processing operation.

### Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

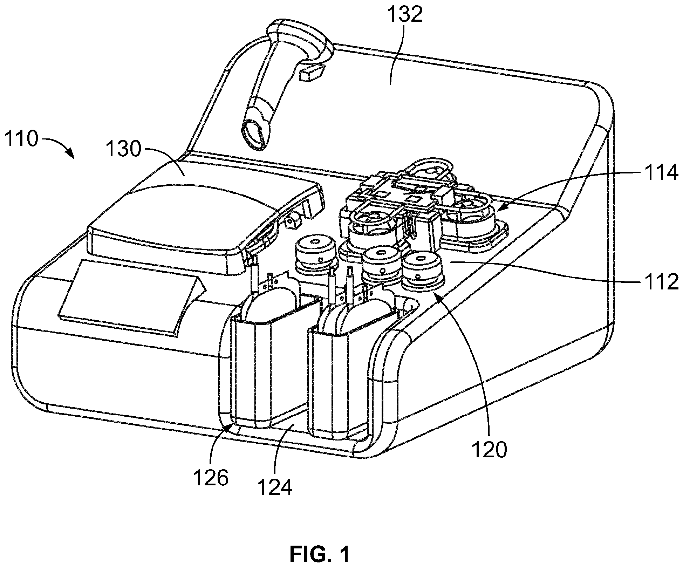

Referring generally to FIGS. 1-8, a blood processing system and/or a blood component collecting or manufacturing system 10 (referred to as a "blood processing system" herein) according to the present examples includes a blood processing device 110, a fluid flow circuit 210 and a packaging tray 310. The packaging tray 310 is configured to accommodate various circuit components of the fluid flow circuit 210 at predetermined positions which correspond to positions of various device components arranged on a component surface 112 of the blood processing device 110. The packaging tray 310 may then be positioned on the blood processing device 110, for example on the component surface 112, such that the various circuit components are aligned and interface with corresponding device components. The blood processing device 110 may then be operated such that the device components interact with corresponding circuit components to control fluid flow through the fluid flow circuit 210 at selected parameters. In this manner, the blood processing device 110 is configured to perform a selected blood processing operation.

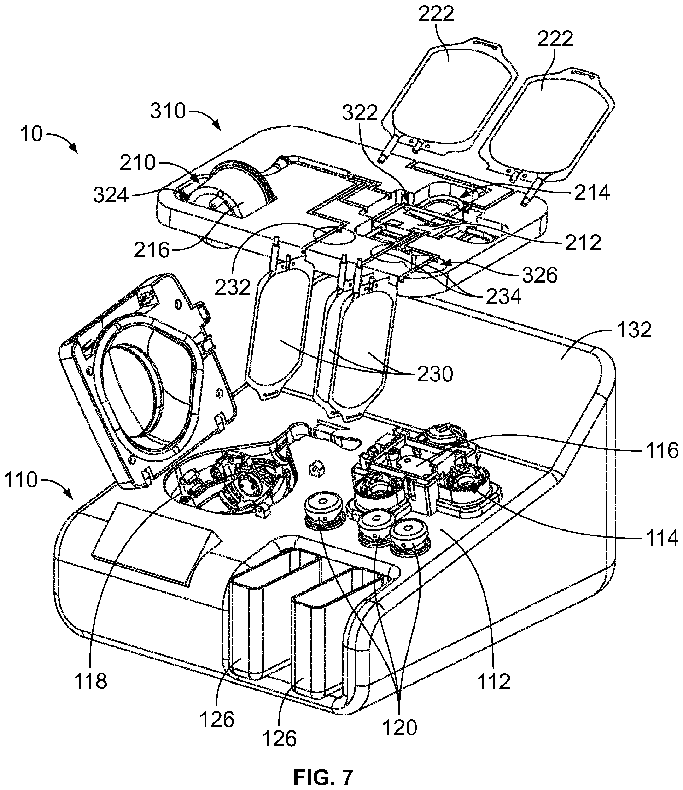

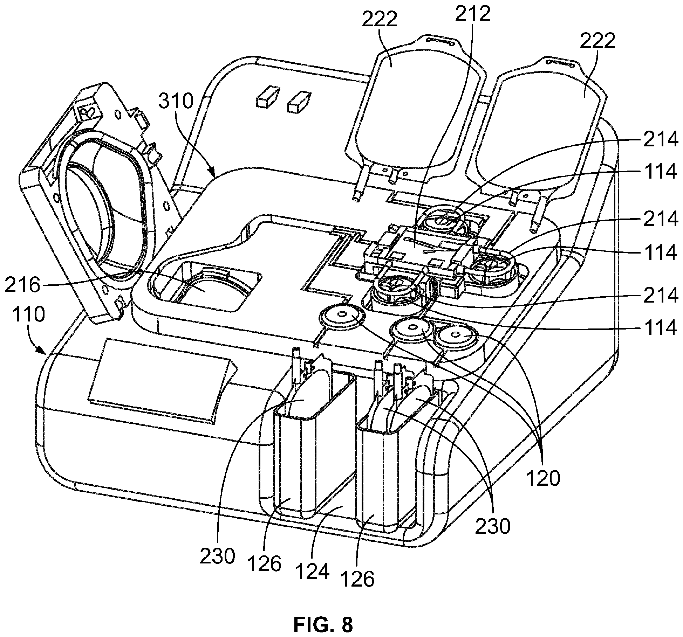

The blood processing device 110 may be a reusable or durable hardware component configured to be used in combination with the fluid flow circuit 210, as shown in FIGS. 7 and 8, for example, and described further below. The fluid flow circuit 210 may be provided as a disposable fluid flow circuit or kit. The blood processing device 110 is configured to interact with the disposable fluid flow circuit 210 to perform a selected blood processing operation. For example, device components may be controlled to operate at preset or selected parameters to control fluid flow (e.g., flow rates, pressures, etc.) through the disposable fluid flow circuit 210 to perform the selected blood processing operation.

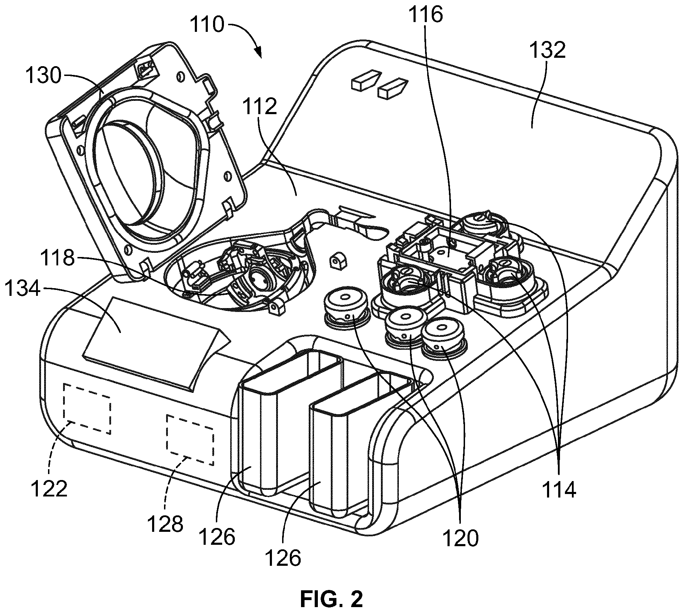

Referring now to FIGS. 1 and 2, the blood processing device 110 includes device components arranged on the component surface 112 and a holder 124 configured to support one or more fluid containers (of the disposable kit). The various device components include a pump system 114 including a plurality of pumps, a cassette nesting module 116, a centrifuge mounting station and drive unit 118, and a plurality of tubing clamps 120. The cassette nesting module 116 may include a plurality of valves and pressure sensors. The blood processing device 110 may also include a controller 122 and a weight scale 128 operably connected to the holder 124 and/or the one or more fluid containers and the controller 122. The weight scale 128 is configured to detect a weight of the holder 124 and/or one or more of the fluid containers supported by the holder 124, and transmit a signal to the controller 122 indicative of the detected weight. The controller 122 is configured to determine a weight of the one or more fluid containers, collectively or individually, based on the transmitted signal indicative of the detected weight. In some examples, multiple holders 124 may be provided, each configured to support one or more fluid containers, and the weight scale 128 may be configured to detect a weight of each holder 124 and/or a weight of the one fluid container(s) supported by each holder 124. A chamber door 130 may selectively cover the centrifuge mounting station and drive unit 118. For example, the chamber door 130 may be in a closed position to cover the centrifuge mounting station and drive unit 118 when the blood processing device 110 is not in use and may be in an open position to allow access to the centrifuge mounting station and drive device 118 when the blood processing device 110 is in use.

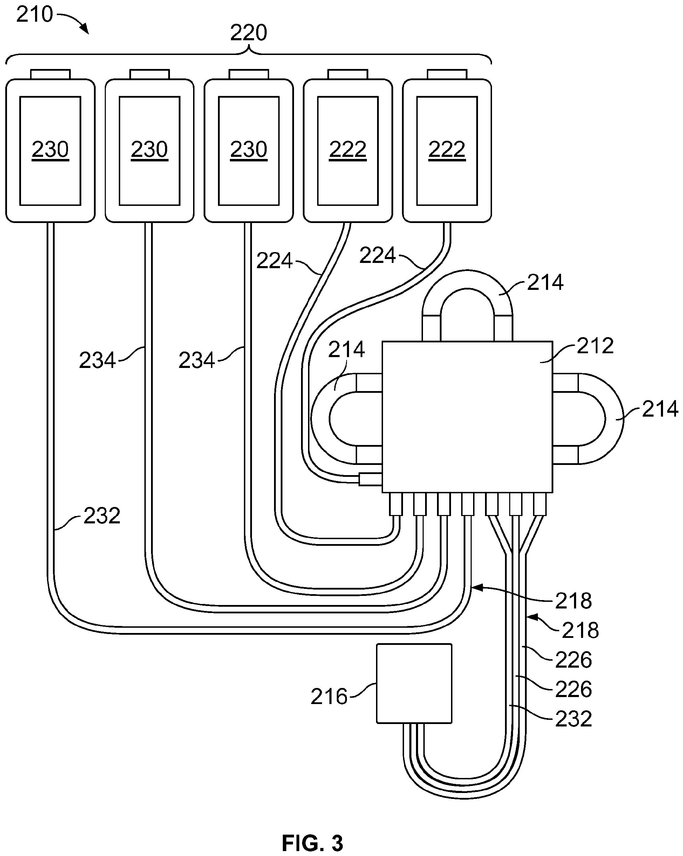

Referring to FIG. 3, the disposable fluid flow circuit or kit 210 includes circuit components configured to interface with corresponding device components on the component surface 112 and interact with the corresponding device components in a selected blood processing operation. The circuit components include a fluid flow control cassette 212 having external tubing loops 214 and configured to be mounted on the cassette nesting module 116, wherein the external tubing loops 214 are engageable with respective pumps of the pump system 114. The circuit components also include a separation chamber 216 configured to be received in the centrifuge mounting station and drive unit 118, and conduit or tubing segments 218, at least portions of which are configured to be received in corresponding tubing clamps 120. The fluid flow circuit 210 may also include fluid containers 220. Conduits or tubing segments 218 may also be provided to fluidically connect the fluid containers 220 to the circuit components such that the circuit components may receive fluid from and/or discharge fluid to the fluid containers 220 via the tubing segments 218. The tubing segments 218 may also extend between the circuit components to fluidically connect the circuit components to one another.

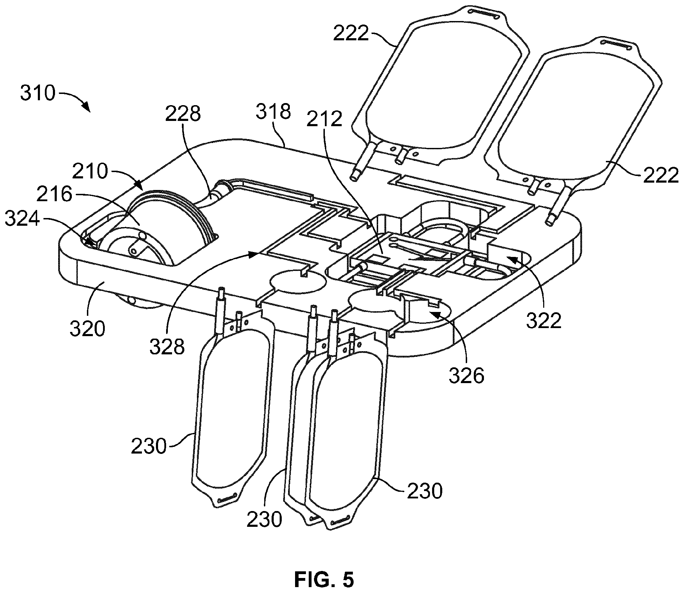

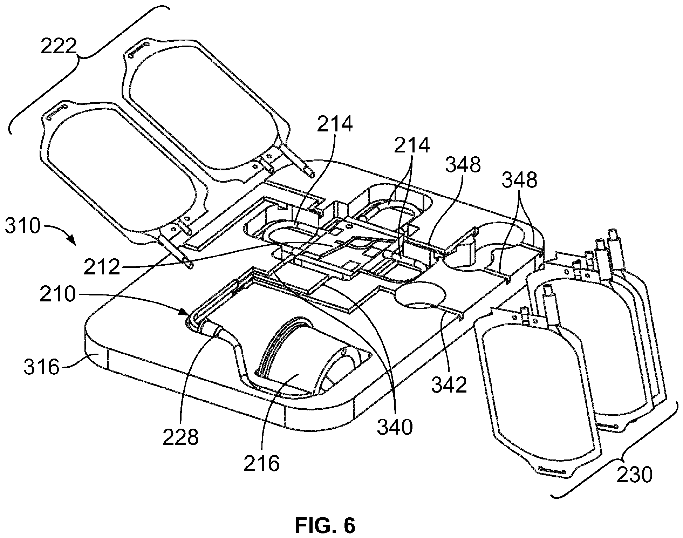

The fluid containers 220 may include one or more first fluid containers 222 and the tubing segments 218 may include one or more first tubing segments 224 to fluidically connect the one or more first fluid containers 222 to the fluid flow control cassette 212. The tubing segments 218 may also include one or more second tubing segments 226 to fluidically connect the fluid flow control cassette 212 to the separation chamber 216. In one example, the separation chamber 216 includes an umbilicus 228 (shown in FIGS. 5 and 6, for example) and the one or more second tubing segments 226 are connected to the umbilicus 228. The fluid containers 220 may further include one or more second fluid containers 230 configured to receive fluid from one or more of the circuit components, and the tubing segments 218 may further include one or more third tubing segments 232 to fluidically connect the separation chamber 216 to a corresponding second fluid container 230. The tubing segments 218 may further include one or more fourth tubing segments 234 to fluidically connect the fluid flow control cassette 212 to corresponding second fluid containers 230 of the second fluid containers 230.

The fluid containers 220 may include, for example, a red blood cell collection container, a plasma collection container, and an additive solution container. The fluid containers 220 may also include a whole blood supply container to supply whole blood for processing. It will be appreciated that varying numbers of fluid containers and types of fluid held in the fluid containers 220 may vary depending on the blood processing operation performed by the blood processing system and/or blood component collecting or manufacturing system 10. In the illustrated examples, the first fluid containers 222 are representative of supply containers, such as the whole blood supply container or the additive solution container referenced above. The second fluid containers 230 are representative of collection containers, such as the red blood cell collection container and plasma collection container referenced above and may also include a buffy coat collection container.

The fluid flow control cassette 212 includes various flow paths either formed integrally on the cassette and/or by conduits connected to the cassette and fluidically connected to the external tubing loops 214. The fluid flow control cassette 212 is mounted on the nesting module 116 and fluid flow through the cassette 212 can be controlled by actuation of the pumps 114 to interact with the external tubing loops 214 and by actuation of the valves to interact with the flow paths of the cassette 212. The pumps 114 may be peristaltic pumps configured to interact with respective tubing loops 214 to cause fluid flow through the tubing loops 214.

The separation chamber 216 can be mounted in the centrifuge mounting station and drive unit 118 (Fig. 2). The centrifuge mounting station and drive unit 118 can be operated at various speeds and time intervals to effect fluid flow and processing in the separation chamber 216.

The tubing segments 218, for example the one or more third tubing segments 232 and one or more fourth tubing segments 234, are received in respective clamps 120 of the plurality of tubing clamps, and the clamps 120 are operable to selectively open and close fluid paths through the tubing segments 232, 234. The clamps 120 may also include RF sealers configured to complete a heat seal of the tubing segment 232, 234 placed in the clamp 120 to seal the tubing segment 232, 234 leading to a fluid or product container (e.g., one or more of the second fluid containers 230) upon completion of a procedure.

Sterile connection/docking devices may also be incorporated into one or more of the clamps 120. The sterile connection devices may employ any of several suitable different operating principles. For example, known sterile connection devices and systems include radiant energy systems that melt facing membranes of fluid flow conduits, heated wafer systems that employ wafers for cutting and heat bonding or splicing tubing segments together while the ends remain molten or semi-molten, and system employing removable closure films or webs sealed to ends of the tubing or conduit segments. Alternatively, sterile connections may be formed by compressing or pinching a sealed tubing segment, heating and severing the sealed end, and joining the tubing to a similarly treated tubing segment. Sterile connection devices based on other operating principles may also be employed without departing from the scope of the present disclosure.

Examples of a blood processing device and disposable fluid flow circuit or kit are shown and described in WO2021/194824 by Fenwal, Inc., which is incorporated herein by reference in its entirety. It will be appreciated that the blood processing device 110 and the disposable fluid flow circuit 210 of the present examples may be largely, but not entirely, the same as the processing device and fluid flow circuit described in WO2021/194824 particularly with respect to components and function, unless described otherwise herein. For example, as described below, while generally including the same components and performing the same or similar functions of the processing device described in WO2021/194824, the blood processing device 110 and/or disposable fluid flow circuit or kit 210 of the present examples may include a modified layout of components and be provided in a more compact form.

Referring again to FIGS. 1 and 2, in the illustrated example, the holder 124 may be provided as one or more cups 124. The cups 124 may be arranged in a receptacle 126 of the blood processing device 110 which is recessed relative to the component surface 112. In one example, one or more second fluid containers 230 are received in the cups 124 (see FIG. 8). In the illustrated example, two cups 124 are shown, one of which contains one second fluid container 230 and the other of which contains two second fluid containers 230. It will be appreciated that the present examples are not limited to the illustrated arrangement of cups and fluid containers however, and that different configurations may be accommodated for different blood processing operations. For example, one or more of the cups 124 may be arranged on a platform or on respective platforms within, or in place of, the receptacle 126. In another example, the holder 124 may be provided as one or more platforms implemented as part of, or connected to, the processing device 110. In still another example, the holder 124 may be provided as a hanger or rack, from which one or more of the fluid containers 220 may be suspended. Such a hanger or rack may extend vertically from the processing device 110, and may be of the type shown and described in WO2021/194824.

The blood processing device 110 may further include a support surface 132 extending from the component surface 112 at an angle relative to the component surface 112. For example, the support surface 132 may extend upward from the component surface 112 and may also be non-planar with the component surface 112. As described further below and shown in FIG. 8, the support surface 132 is configured to support, for example, one or more peripheral devices of the blood processing device 110, such as a scanner, and/or one or more fluid containers 220 of the fluid flow circuit 210, such as the first fluid containers 222.

The blood processing device 110 may be provided with a user interface 134 configured to receive information (i.e., instructions) from an operator and/or output information regarding operation of the blood processing device 110 to the operator. The user interface 134 is operably connected to the controller 122, or alternatively, is integrated with the controller 122. In use, the user interface 134 is configured to receive an operator instruction and the controller 122 is configured to control one or more components of the blood processing device 110 according to the operator instruction. In one example, the user interface 134 may include a touch screen display. Alternatively, or in addition, the user interface 134 may include one or more of a switch, knob, dial, microphone, sensor (e.g., biometric, optical, etc.), scanner (e.g., QR code, bar code, etc.) or other suitable interface for receiving a user instruction.

The user interface 134 may also be configured to output information, for example, regarding a blood processing operation performed by the blood processing device 110. In one example, the user interface 134 may display the information on the touch screen display. Alternatively, or in addition, the user interface 134 may output information using other devices, such as an audio speaker, one or more lights, and the like. Such information may include one or more various parameters of the blood processing operation, for example, a temperature of the whole blood and/or whole blood components, the weight of fluid containers used in the blood processing operations, status of the operation, as well as operating parameters of the blood processing device 110, i.e., the parameters at which the device 110 is controlled to operate to perform a selected blood processing operation.

Further, the controller 122 may include, or be operably connected to, wireless communication capabilities to enable transfer of data from the blood processing device 110 to, for example, a quality management system and/or other operator system.

The controller 122 may be pre-programmed to automatically operate the blood processing device 110 to perform one or more standard blood processing operations selected by an operator, for example, at the user interface 134. The controller 122 may also be programmed by the operator to perform additional and/or non-standard blood processing procedures. For example, the controller 122 may be pre-programmed to substantially automate a wide variety of blood processing operations, including, but not limited to: red blood cell and plasma production from a single unit of whole blood, buffy coat pooling, buffy coat separation into a platelet product (as described in U.S. Patent Application Publication No. 2018/0078582, which is hereby incorporated herein by reference in its entirety), glycerol addition to red blood cells, red blood cell washing, platelet washing, and cryoprecipitate pooling and separation.

The pre-programmed blood processing operations are performed by the blood processing device 110 at preset settings for flow rates and centrifugation forces, and the controller 122 may be further configured to receive input (i.e., instructions) from the operator as to one or more of flow rates and centrifugation forces for the standard blood processing procedure to override the pre-programmed settings.

In the examples above, the controller 122 may include a programmable microprocessor and a memory coupled to the microprocessor. The memory includes a non-transitory computer readable medium and is configured to store program instructions to be executed by the microprocessor. The microprocessor is configured to perform one or more operations in response to executing the program instructions. For example, the microprocessor may control various components (e.g., pumps 114, valves of nesting module 116, centrifuge mounting station and drive unit 118, clamps 120, etc.) to perform a selected blood processing operation based on the program instructions associated with such an operation. The memory may also store the preset settings for blood processing operations as well as information detected by various sensors relating to, for example, operating and/or environmental parameters of the blood processing device 110 and/or fluid circuit 210.

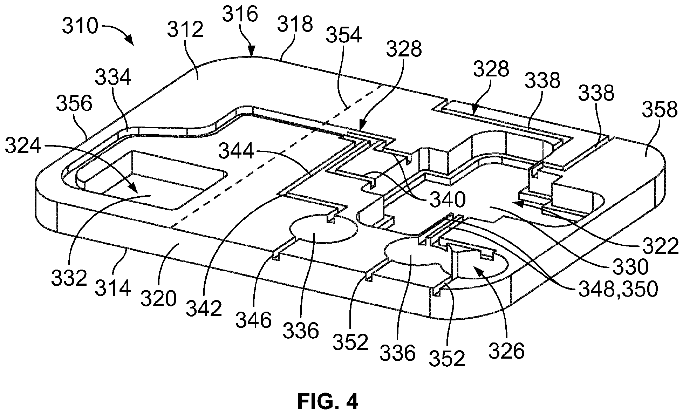

Referring now to FIGS. 4-6, the blood processing system 10 further includes the packaging tray 310. The packaging tray 310 is configured for use in a blood processing system including a blood processing device having device components arranged on a component surface and removable fluid flow circuit components configured to interact with corresponding device components. The packaging tray 310 is also configured to be selectively arranged on and removable from the component surface. Further, the packaging tray 310 may be configured to accommodate circuit components of the fluid flow circuit 210 at positions aligned with corresponding device components of the blood processing device 110. In this manner, circuit components of the fluid flow circuit 210 may be arranged at designated positions on the packaging tray 310, and the packaging tray 310 may be disposed on the component surface 112 of the blood processing device 110 to position the circuit components relative to corresponding device components for performing a blood processing operation. In some examples, the packaging tray 310 may be considered a component of the blood processing device 110 configured for selective arrangement on and removal from the component surface 112. In other examples, the packaging tray 310 may be provided separately from the blood processing device 310.

The packaging tray 310 includes a first surface 312 and a second surface 314 opposite to the first surface 312 and spaced apart by a thickness. The packaging tray 310 also includes component sections arranged at positions corresponding to positions of respective device components on the component surface 112 at which device components are arranged. That is, the component sections of the packaging tray 310 are positioned on the packaging tray 310 and spaced from another to align with positions of corresponding device components on the component surface 112. The component sections may be respectively formed, at least partially, as openings extending through the thickness. The component sections may also be configured to receive at least a portion of one or more of the device components when the packaging tray 310 is arranged on the blood processing device 110. It will be appreciated that the packaging tray 310 may be manufactured having component sections differing in number, shape, position and/or size from the illustrated examples. In this manner, the packaging tray 310 may be manufactured for use with blood processing devices and/or component surfaces having differently arranged device components.

In one example, the first surface 312 is configured to accommodate the circuit components and the second surface 314 is configured to face the component surface 112. A thickness of the packaging tray 310 is formed between the first surface 312 and the second surface 314 such that the first surface 312 and second surface 314 are spaced apart by the thickness. The packaging tray 310 also includes a peripheral edge 316 which includes at least a first peripheral edge 318 and a second peripheral edge 320.

The various component sections may include, for example, a first component section 322 configured to accommodate the fluid flow control cassette 212 and external tubing loops 214, a second component section 324 configured to accommodate the separation chamber 216 and a third component section 326 configured to accommodate at least a portion of the tubing segments 218. The packaging tray 310 may further include various pathways 328 configured to receive portions of the tubing segments 218. The pathways 328 may be recessed on the first surface 312.

The first component section 322 may be formed, at least partially, as a first opening 330 extending through the thickness of the packaging tray 310 i.e., from the first surface 312 to the second surface 314. The first component section 322 may be sized and shaped to receive at least a portion of the fluid flow control cassette 212 and external tubing loops 214. The first component section 322 may also be sized and shaped to receive at least a portion of the pumps 114 and the nesting module 116, for example, in the first opening 330.

A second component section 324 is spaced from the first component section 322 and may be formed, at least partially, as a second opening 332 extending through the thickness of the packaging tray 310. The second component section 324 may be sized and shaped to receive at least a portion of the separation chamber 216. The second component section 324 may also include a recessed or grooved portion 334 connected to the second opening 332 configured to receive the umbilicus 228.

A third component section 326 is spaced from the first and second component sections 322, 324 and may be formed, at least partially, as one or more third openings 336 extending through the thickness of the packaging tray 310. The third component section 326 may be sized and shaped to receive at least a portion of the tubing segments 218, such as portions of the third and fourth tubing segments 232, 234. The third component section 326 may also be sized and shaped to receive at least a portion of the clamps 120 arranged on the component surface 112, for example, in the one or more third openings 336.

The packaging tray 310 also includes the pathways 328 configured to accommodate corresponding tubing segments 218 on the first surface 312. The pathways 328 may extend between, be connected to, and/or intersect one or more of the peripheral edge 316, the first component section 322, the second component section 324, and/or the third component section 326. In some examples, pathways 328 may intersect the first peripheral edge 318 and/or the second peripheral edge 320.

In the illustrated example, the pathways 328 may include one or more first pathways 338 extending between the peripheral edge 316, for example the first peripheral edge 318, and the first component section 322. The one or more first pathways 338 are configured to accommodate the corresponding one or more first tubing segments 224 which fluidically connect first fluid containers 222 to the flow control cassette 212.

The pathways 328 may further include one or more second pathways 340 extending between the first component section 322 and the second component section 324. The one or more second pathways 340 are configured to accommodate the corresponding one or more second tubing segments 226 which fluidically connect the flow control cassette 212 to the separation chamber 216.

The pathways 328 may further include one or more third pathways 342 extending between the second component section 324 and the peripheral edge 316, for example, the second peripheral edge 320. The one or more third pathways 342 are configured to accommodate the corresponding one or more third tubing segment 232 between the separation chamber 216 and a corresponding second fluid container 230.

In one example, the one or more third pathway 342 intersects the third component section 326 and includes a first portion 344 extending between the second component section 324 and the third component section 326 and a second portion 346 extending between the third component section 326 and the second peripheral edge 320. In this manner, the third tubing segment 232 arranged in the corresponding third pathway 342 may be received in a corresponding clamp 120 at the third component section 326.

The pathways 328 may further include one or more fourth pathways 348 extending between the first component section 322 and the peripheral edge 316. For example, the second peripheral edge 320. The one or more fourth pathways 348 are configured to accommodate the corresponding one or more fourth tubing segments 234 between the flow control cassette 212 and a corresponding second fluid container 230.

In one example, the one or more fourth pathways 348 intersect the third component section 326 and include a first portion 350 extending between the first component section 322 and the third component section 326 and a second portion 352 extending between the third component section 326 and the second peripheral edge 320. In this manner, the fourth tubing segments 234 arranged in corresponding fourth pathways 348 may be received in corresponding clamps 120 at the third component section 326.

The packaging tray 310 may include a folding line 354 along which the packaging tray 310 may be folded. Accordingly, when the packaging tray 310 is arranged on the blood processing device 110, a portion of the packaging tray 310 may be folded away from the component surface 112 to allow access to the chamber door 130 over the centrifuge mounting station and drive unit 118. The folding line 354 may be a pre-weakened section formed in the material of the packaging tray 310. For example, the folding line 354 may be a perforated line.

In one example, the folding line 354 extends generally between opposite edges of the peripheral edge 316, for example, between the first peripheral edge 318 and the second peripheral edge 320. The packaging tray 310 may include a first tray portion 356 on one side of the folding line 354 and a second tray portion 358 on another side of the folding line 354, wherein the folding line 354 allows the first tray portion 356 to fold relative to the second tray portion 358. In the illustrated example, the second component section 324 is located on the first tray portion 356 and the first and third component sections 322, 326 are located on the second portion 358.

Referring to FIGS. 5 and 6, the fluid flow circuit 210 may be arranged on the packaging tray 310 with the flow control cassette 212 positioned at the first component section 322, the separation chamber 216 positioned at the second component section 324 and the tubing segments 218 (omitted from FIGS. 5 and 6) positioned in corresponding pathways 328. Corresponding tube segments 218, such as the third and fourth tubing segments 232, 234 may also intersect and be positioned in the third component section 326. The fluid containers 220 may be arranged at or near the peripheral edge 316. For example, the first fluid containers 222 may be arranged adjacent to the first peripheral edge 318 and the second fluid containers 230 may be arranged adjacent to the second peripheral edge 320.

With reference to FIG. 7, the packaging tray 310, with the fluid flow circuit 210 arranged thereon, may be positioned relative to the blood processing device 110 such that the flow control cassette 212, external tubing loops 214 and first component section 322 are aligned with the pump system 114 and nesting module 116, the separation chamber 216 and second component section 324 are aligned with the centrifuge mounting station and drive unit 118, and the corresponding tubing segments (e.g., third and fourth tubing segments 232, 234) and the third component section 326 are aligned with the clamps 120. In addition, one or more of the first fluid containers 222 may be aligned with the support surface 132 and one or more of the second fluid containers 230 may be aligned with the holder 124.

Referring now to FIGS. 7 and 8, the packaging tray 310, with the fluid flow circuit 210 arranged thereon, may be positioned on the blood processing device 110 and the circuit components are aligned and interfaced with corresponding device components. Accordingly, the blood processing device 110 may be operated such that the device components interact with corresponding circuit components to perform a selected blood processing operation.

For example, as referenced above, the flow control cassette 212 is mounted on the nesting module 116 and the valves of the nesting module 116 may be operated to control fluid flow through flow paths of the flow control cassette 212. The external tubing loops 214 engage corresponding pumps 114 and the pumps 114 may be operated to cause fluid flow through the external tubing loops 214. The separation chamber 216 is mounted in the centrifuge mounting station and drive unit 118 and the centrifuge mounting station and drive unit 118 may be operated to cause flow, centrifugation and/or processing of fluid within the separation chamber 216. Further still, the tubing segments 218, e.g., the third and fourth tubing segments 232, 234 (not shown in FIG. 8) are at least partially received in corresponding clamps 120 and the clamps 120 may be operated to selectively control fluid flow through tubing segments 232, 234. The clamps 120 may also be operated to seal corresponding tubing segments 232, 234.

Accordingly, in the manner above, circuit components 212, 214, 216, 218 of the fluid flow circuit or kit 210 can be arranged on the packaging tray 310 at corresponding component sections 322, 324, 326 and pathways 328 by an operator in a relatively convenient manner. The packaging tray 310 can then be arranged on the blood processing device 110 with the circuit components 212, 214, 216, 218 aligned and interfaced with corresponding device components 114, 116, 118, 120 to perform a blood processing operation. Thus, an operator may avoid individually and sequentially mounting each circuit component 212, 214, 216, 218 on a corresponding device component 114, 116, 118, 120.

In addition, with a relatively compact configuration of the blood processing device 110, various operator movements (e.g., vertical reach or extension) may be limited or reduced when arranging the fluid flow circuit 210 on the blood processing device 110 using the packaging tray 310. For example, an operator may position one or more of the fluid containers 220 adjacent to the peripheral edge 316 of the packaging tray 310 to be supported on the support surface 132 or by the holder 124 of the blood processing device 110, and thus, may avoid movements required to hang or suspend fluid containers at elevated positions on a vertical hanger or rack.

### Aspects

Aspect 1. A packaging tray for use in a blood processing system comprising a blood processing device having device components arranged on a component surface and a removable fluid flow circuit having circuit components configured to interact with corresponding device components, the packaging tray configured to be selectively arranged on and removable from the component surface, the packaging tray comprising: a first surface and a second surface opposite to the first surface and spaced apart by a thickness; component sections arranged at positions corresponding to positions of respective device components, the component sections respectively formed, at least partially, as openings extending through the thickness; and a plurality of recessed pathways formed on the first surface, the recessed pathways extending between the component sections.

Aspect 2. The packaging tray of Aspect 1, the component sections comprising: a first component section formed at least partially as a first opening extending through the thickness; a second component section spaced from the first component section, the second component section formed at least partially as a second opening extending through the thickness; and a third component section spaced from the first and second component sections, the third component section formed at least partially as one or more third openings extending through the thickness, wherein the recessed pathways extend between the first component section, the second component section and the third component section.

Aspect 3. The packaging tray of Aspect 2, further comprising a peripheral edge and wherein the plurality of recessed pathways comprise: one or more first pathways extending between the peripheral edge and the first component section; one or more second pathways extending between the first component section and the second component section; one or more third pathways extending between the third component section and the peripheral edge, wherein the one or more third pathways intersect the third component section; and one or more fourth pathways extending between the first component section and the peripheral edge, wherein the one or more fourth pathways intersect the third component section.

Aspect 4. The packaging tray of Aspect 3, wherein the peripheral edge includes a first peripheral edge and a second peripheral edge opposite to the first peripheral edge, the packaging tray further comprising: a folding line extending between the first peripheral edge and the second peripheral edge; and a first tray portion on one side of the folding line and a second tray portion on another side of the folding line, wherein the first tray portion is foldable relative to the second tray portion along the folding line.

Aspect 5. A blood processing device comprising: device components arranged on a component surface; a holder configured to support one or more fluid containers; and a packaging tray configured to be selectively arranged on and removable from the component surface, the packaging tray comprising: a first surface and a second surface opposite to the first surface and spaced apart by a thickness; component sections arranged at positions corresponding to positions of respective device components, the component sections respectively formed, at least partially, as openings extending through the thickness; and a plurality of recessed pathways formed on the first surface, the recessed pathways extending between the component sections.

Aspect 6. The blood processing device of Aspect 5, wherein the device components include a nesting module and pump system, a centrifuge mounting station and drive unit, and a plurality of clamps, and wherein the component sections include a first component section aligned with the nesting module and pump system, a second component section aligned with the centrifuge mounting station and drive unit, and a third component section aligned with the plurality of clamps with the packaging tray arranged on the component surface.

Aspect 7. The blood processing device of Aspect 6, wherein the packaging tray further comprises a folding line allowing a first tray portion to fold relative to a second tray portion to expose a portion of the component surface.

Aspect 8. The blood processing device of Aspect 7, wherein the second component section is located on the first tray portion and the first and third component sections are located on the second tray portion.

Aspect 9. The blood processing device of any of Aspects 6-8, further comprising: a support surface extending upward from and angled relative to the component surface, wherein the support surface is non-planar relative to the component surface; a controller operably connected to and configured to control the pump system, the cassette nesting module, the centrifuge mounting station and drive unit and the plurality of tubing clamps; a user interface operably connected to the controller, wherein the user interface is configured to receive an instruction from an operator and the controller is configured to selectively control one or more of the pump system, the cassette nesting module, the centrifuge mounting station and drive unit and the plurality of tubing clamps according to the instruction; and a weight scale operably connected to the holder, wherein the weight scale is configured to detect a weight of the holder and/or the one or more fluid containers and transmit a signal to the controller indicative of the detected weight.

Aspect 10. The blood processing device of Aspect 9, wherein the holder includes at least one cup configured to hold the one or more fluid containers and the controller is configured to determine a weight of the one or more fluid containers based on the signal indicative of the detected weight.

Aspect 11. A blood processing system comprising: a blood processing device having device components arranged on a component surface and a holder configured to support one or more fluid containers; a packaging tray configured to be selectively arranged on and removable from the component surface, the packaging tray comprising: a first surface and a second surface opposite to the first surface and spaced apart by a thickness, component sections arranged at positions corresponding to positions of respective device components, the component sections respectively formed, at least partially, as openings extending through the thickness, and a plurality of recessed pathways formed on the first surface, the recessed pathways extending between the component sections; and a fluid flow circuit having circuit components configured to interact with corresponding device components, wherein the one or more fluid containers are arrangable in fluid communication with the circuit components, wherein the component sections are configured to accommodate corresponding circuit components such that the circuit components are interfaced with corresponding device components with the packaging tray arranged on the component surface.

Aspect 12. The system of Aspect 11, wherein: the device components include a nesting module and pump system, a centrifuge mounting station and drive unit, and a plurality of clamps, the circuit components include a flow control cassette and external tubing loops, a separation chamber, and tubing segments, and the flow control cassette and external tubing loops are configured to interact with the nesting module and pump system, the separation chamber is configured to interact with the centrifuge mounting station and drive unit, and at least a portion of the tubing segments are configured to interact with the plurality of clamps.

Aspect 13. The system of Aspect 12, wherein the component sections include: a first component section aligned with the nesting module and pump system and configured to accommodate the flow control cassette and external tubing loops, a second component section aligned with the centrifuge mounting station and drive unit and configured to accommodate the separation chamber, and a third component section aligned with the plurality of clamps and configured to accommodate the at least a portion of the tubing segments.

Aspect 14. The system of Aspect 13, wherein the fluid containers include one or more first fluid containers and one or more second fluid containers, and wherein the tubing segments comprise: one or more first tubing segments connected between the first fluid containers and the flow control cassette, one or more second tubing segments connected between the flow control cassette and the separation chamber, one or more third tubing segments connected between the separation chamber and a corresponding second fluid container of the one or more second fluid containers, and one or more fourth tubing segments connected between the flow control cassette and corresponding second fluid containers of the one or more second fluid containers.

Aspect 15. The system of Aspect 14, wherein the plurality of recessed pathways include: one or more first pathways extending between a peripheral edge of the packaging tray and the first component section and configured to accommodate the corresponding one or more first tubing segments; one or more second pathways extending between the first component section and the second component section and configured to accommodate the corresponding one or more second tubing segments; one or more third pathways extending between the third component section and the peripheral edge and configured to accommodate the corresponding one or more third tubing segment; and one or more fourth pathways extending between the first component section and the peripheral edge and configured to accommodate the corresponding one or more fourth tubing segments.

Aspect 16. The system of Aspect 15, wherein the one or more third pathway intersects the third component section and includes a first portion extending between the second component section and the third component section and a second portion extending between the third component section and the peripheral edge, and wherein the one or more fourth pathways intersect the third component section and include a first portion extending between the first component section and the third component section and a second portion extending between the third component section and peripheral edge.

Aspect 17. The system of any of Aspects 14-16, wherein the blood processing device further comprises a support surface extending from the component surface at an angle relative to the component surface, wherein the support surface is configured to support at least one of the one or more first fluid containers, and the holder is configured to support at least one of the one or more second fluid containers.

Aspect 18. The system of any of Aspects 11-17, wherein the blood processing device further comprises a controller and a user interface operably connected to the controller, wherein the user interface is configured to receive a user instruction and the controller is configured to operate one or more of the device components based on the user instruction.

Aspect 19. The system of Aspect 18, wherein the blood processing device further comprises a weight scale operably connected to the holder and the controller, wherein the holder is configured to support one or more of the fluid containers and the weight scale is configured to detect a weight of the holder and/or the one or more fluid containers supported by the holder and transmit a signal to the controller indicative of the detected weight.

Aspect 20. The system of any of Aspects 11-19, wherein the packaging tray further comprises a folding line allowing a first tray portion to fold relative to a second tray portion.
It will be understood that the embodiments and examples described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A packaging tray for use in a blood processing system comprising a blood processing device having device components arranged on a component surface and a removable fluid flow circuit having circuit components configured to interact corresponding device components, the packaging tray configured to be selectively arranged on and removable from the component surface, the packaging tray comprising:
a first surface and a second surface opposite to the first surface and spaced apart by a thickness;
component sections arranged at positions corresponding to positions of respective device components, the component sections respectively formed, at least partially, as openings extending through the thickness; and
a plurality of recessed pathways formed on the first surface, the recessed pathways extending between the component sections.

2. The packaging tray of claim 1, the component sections comprising:
a first component section formed at least partially as a first opening extending through the thickness;
a second component section spaced from the first component section, the second component section formed at least partially as a second opening extending through the thickness; and
a third component section spaced from the first and second component sections, the third component section formed at least partially as one or more third openings extending through the thickness,
wherein the recessed pathways extend between the first component section, the second component section and the third component section.

3. The packaging tray of claim 1, further comprising a peripheral edge and wherein the plurality of recessed pathways comprise:
one or more first pathways extending between the peripheral edge and the first component section;
one or more second pathways extending between the first component section and the second component section;
one or more third pathways extending between the third component section and the peripheral edge, wherein the one or more third pathways intersect the third component section; and
one or more fourth pathways extending between the first component section and the peripheral edge, wherein the one or more fourth pathways intersect the third component section.

4. The packaging tray of claim 3, wherein the peripheral edge includes a first peripheral edge and a second peripheral edge opposite to the first peripheral edge, the packaging tray further comprising:
a folding line extending between the first peripheral edge and the second peripheral edge; and
a first tray portion on one side of the folding line and a second tray portion on another side of the folding line, wherein the second component section is located on the first tray portion and the first and third component sections are located on the second tray portion,
wherein the first tray portion is foldable relative to the second tray portion along the folding line.

5. A blood processing device comprising:
device components arranged on a component surface;
a holder configured to support one or more fluid containers; and
a packaging tray configured to be selectively arranged on and removable from the component surface, the packaging tray comprising:
a first surface and a second surface opposite to the first surface and spaced apart by a thickness;
component sections arranged at positions corresponding to positions of respective device components, the component sections respectively formed, at least partially, as openings extending through the thickness; and
a plurality of recessed pathways formed on the first surface, the recessed pathways extending between the component sections.

6. The blood processing device of claim 5, wherein the device components include a nesting module and pump system, a centrifuge mounting station and drive unit, and a plurality of clamps, and
wherein the component sections include a first component section aligned with the nesting module and pump system, a second component section aligned with the centrifuge mounting station and drive unit, and a third component section aligned with the plurality of clamps with the packaging tray arranged on the component surface.

7. The blood processing device of claim 5, further comprising:
a support surface extending upward from and angled relative to the component surface, wherein the support surface is non-planar relative to the component surface;
a controller operably connected to and configured to control the pump system, the cassette nesting module, the centrifuge mounting station and drive unit and the plurality of tubing clamps;
a user interface operably connected to the controller, wherein the user interface is configured to receive an instruction from an operator and the controller is configured to selectively control one or more of the pump system, the cassette nesting module, the centrifuge mounting station and drive unit and the plurality of tubing clamps according to the instruction; and
a weight scale connected to the holder, wherein the weight scale is configured to detect a weight of the holder and/or the one or more fluid containers and transmit a signal to the controller indicative of the detected weight.

8. The blood processing device of claim 7, wherein the holder includes at least one cup configured to hold the one or more fluid containers and the controller is configured to determine a weight of the one or more fluid containers based on the signal indicative of the detected weight.

9. A blood processing system comprising:
a blood processing device having device components arranged on a component surface and a holder configured to support one or more fluid containers;
a packaging tray configured to be selectively arranged on and removable from the component surface, the packaging tray comprising:
a first surface and a second surface opposite to the first surface and spaced apart by a thickness,
component sections arranged at positions corresponding to positions of respective device components, the component sections respectively formed, at least partially, as openings extending through the thickness, and
a plurality of recessed pathways formed on the first surface, the recessed pathways extending between the component sections; and
a fluid flow circuit having circuit components configured to interact with corresponding device components, wherein the one or more fluid containers are arranged in fluid communication with the circuit components,
wherein the component sections are configured to accommodate corresponding circuit components such that the circuit components are interfaced with corresponding device components with the packaging tray arranged on the component surface.

10. The system of claim 9, wherein:
the device components include a nesting module and pump system, a centrifuge mounting station and drive unit, and a plurality of clamps,
the circuit components include a flow control cassette and external tubing loops, a separation chamber, and tubing segments, and
the flow control cassette and external tubing loops are configured to interact with the nesting module and pump system, the separation chamber is configured to interact with the centrifuge mounting station and drive unit, and at least a portion of the tubing segments are configured to interact with the plurality of clamps.

11. The system of claim 10, wherein the component sections include:
a first component section aligned with the nesting module and pump system and configured to accommodate the flow control cassette and external tubing loops,
a second component section aligned with the centrifuge mounting station and drive unit and configured to accommodate the separation chamber, and
a third component section aligned with the plurality of clamps and configured to accommodate the at least a portion of the tubing segments.

12. The system of claim 11, wherein the fluid containers include one or more first fluid containers and one or more second fluid containers, and wherein the tubing segments comprise:
one or more first tubing segments connected between the first fluid containers and the flow control cassette,
one or more second tubing segments connected between the flow control cassette and the separation chamber,
one or more third tubing segments connected between the separation chamber and a corresponding second fluid container of the one or more second fluid containers, and
one or more fourth tubing segments connected between the flow control cassette and corresponding second fluid containers of the one or more second fluid containers.

13. The system of claim 12, wherein the plurality of recessed pathways include:
one or more first pathways extending between a peripheral edge of the packaging tray and the first component section and configured to accommodate the corresponding one or more first tubing segments;
one or more second pathways extending between the first component section and the second component section and configured to accommodate the corresponding one or more second tubing segments;
one or more third pathways extending between the third component section and the peripheral edge and configured to accommodate the corresponding one or more third tubing segment; and
one or more fourth pathways extending between the first component section and the peripheral edge and configured to accommodate the corresponding one or more fourth tubing segments.

14. The system of claim 13, wherein the one or more third pathway intersects the third component section and includes a first portion extending between the second component section and the third component section and a second portion extending between the third component section and the peripheral edge, and
wherein the one or more fourth pathways intersect the third component section and include a first portion extending between the first component section and the third component section and a second portion extending between the third component section and peripheral edge.

15. The system of claim 9, wherein the blood processing device further comprises a controller and a user interface operably connected to the controller, wherein the user interface is configured to receive a user instruction and the controller is configured to operate one or more of the device components based on the user instruction.
